(19) Europäisches Patentamt
European Patent Office
Office européen des brevets

(11) Veröffentlichungsnummer: **0 049 355**
**B1**

(12) # EUROPÄISCHE PATENTSCHRIFT

(45) Veröffentlichungstag der Patentschrift: **10.10.84**

(21) Anmeldenummer: **81106511.9**

(22) Anmeldetag: **21.08.81**

(51) Int. Cl.³: **C 07 D 471/04,** C 07 D 215/56, A 61 K 31/44, A 61 K 31/445, A 61 K 31/47, A 61 K 31/495, A 61 K 31/535 // (C07D471/04, 221/00, 221/00)

(54) **7-Amino-1-cyclopropyl-4-oxo-1,4-dihydronaphthyridin-3-carbonsäuren, Verfahren zu ihrer Herstellung und sie enthaltende Arzneimittel.**

(30) Priorität: **03.09.80 DE 3033157**

(43) Veröffentlichungstag der Anmeldung: **14.04.82 Patentblatt 82/15**

(45) Bekanntmachung des Hinweises auf die Patenterteilung: **10.10.84 Patentblatt 84/41**

(84) Benannte Vertragsstaaten: **AT BE CH DE FR GB IT LI NL SE**

(56) Entgegenhaltungen:
**EP - A - 0 004 279**
**EP - A - 0 014 390**
**DE - A - 2 840 910**

**Schröder, Arzneimittelchemie, I, S. 33**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Grohe, Klaus, Dr., Am Wasserturm 10, D-5068 Odenthal (DE)**
Erfinder: **Zeiler, Hans-Joachim, Dr., Windrather Strasse 188, D-5620 Velbert 15 (DE)**
Erfinder: **Metzger, Karl Georg, Dr., Pahlkestrasse 15, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft neue 7-Amino-1-cyclopropyl-4-oxo-1,4-dihydro-naphthyridin-3-carbonsäuren, Verfahren zu ihrer Herstellung sowie diese enthaltende Arzneimittel, insbesondere antibakterielle Mittel, sowie Futterzusatzmittel.

Es ist bereits bekanntgeworden, daß 7-Amino-1-ethyl-4-oxo-1,4-dihydro-naphthyridin-3-carbonsäuren antibakterielle Eigenschaften besitzen [Eur. J. Med. Chem. 12, 541—547 (1977)]. Aus DE-A1-2 840 910, EP-A1-0 004 279 und EP-A-0 014 393 sind Verbindungen ähnlicher Struktur bekannt, die ebenfalls antibakteriell wirksam sind. Es wurde nun gefunden, daß die neuen 7-Amino-1-cyclopropyl-4-oxo-1,4-dihydro-naphthyridin-3-carbonsäuren der Formel I

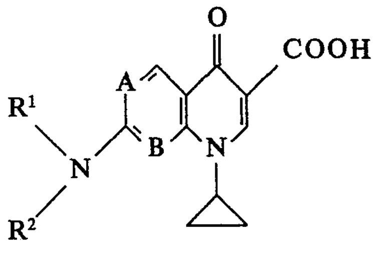

(I)

in welcher

A Stickstoff oder $CR^3$ sein kann, wobei $R^3$ Wasserstoff, Nitro, Halogen, bevorzugt Fluor oder Chlor, oder eine Nitril- Cabonamid, Carboxyl- oder Estergruppe sein kann und

B Stickstoff oder C—H darstellt und A und B nicht gleichzeitig Stickstoff sein können sowie

$R^1$ und $R^2$ gleich oder verschieden sind und für Wasserstoff, einen verzweigten oder unverzweigten Alkyl-, Alkenyl- oder Alkinylrest mit 1—12 Kohlenstoffatomen stehen, der gegebenenfalls durch Hydroxylgruppen, Alkoxy-, Alkylmercapto- oder Dialkyl-amino-gruppen mit 1—3 Kohlenstoffatomen in jedem Alkylrest, die Nitril- sowie eine Alkoxycarbonyl-Gruppe mit 1—4 Kohlenstoffatomen im Alkoholteil substituiert sein kann, ferner Cycloalkyl mit 3—6 Kohlenstoffatomen bedeutet und weiterhin gemeinsam mit dem Stickstoffatom, das sie substituieren und gegebenenfalls einem Heteroatom wie z. B. Sauerstoff, Schwefel oder $NR^4$ einen 3—7gliedrigen Ring bilden, der ein- oder mehrfach durch Alkyl- oder Alkenylgruppen mit 1—6 Kohlenstoffatomen, Hydroxyl-, Alkoxy- und Alkylmercaptogruppen mit 1—3 Kohlenstoffatomen, eine Alkoxycarbonylgruppe mit 1—4 Kohlenstoffatomen im Alkoholteil, die Nitrilgruppe sowie einen Phenylrest substituiert sein kann und ferner eine Doppelbindung besitzen kann und

$R^4$ Wasserstoff, eine verzweigte oder unverzweigte Alkyl-, Alkenyl- oder Alkinylgruppe mit 1—6 Kohlenstoffatomen, die gegebenenfalls durch Hydroxyl, Alkoxy-, Alkylmercapto- und Dialkylaminogruppe mit 1—3 Kohlenstoffatomen für einen Alkylrest, die Alkoxycarbonylgruppe mit 1—4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, sowie eine gegebenenfalls substituierte Phenyl- oder Naphthylgruppe oder ein heterocyclischer Rest wie beispielsweise einen Pyridin-, Pyrimidin-, Thiazol-, oder Benzthiazolkern darstellt, ferner eine gegebenenfalls durch einen Phenylrest substituierte Alkoxycarbonylgruppe mit 1—4 Kohlenstoffatomen im Alkoholteil ein Alkanoylrest mit 1—6 Kohlenstoffatomen, ein Benzoylrest, ein gegebenenfalls substituierter C1—C6-Alkyl- oder Phenylsulfonylrest sowie ein gegebenenfalls substituierter Aminosulfonylrest bedeutet, wobei als Substituenten von Phenyl- und Naphthylgruppen Halogen, Alkyl-, Alkoxy- oder Alkylenmercaptogruppen mit 1—3 Kohlenstoffatomen, Phenyloxy-, Phenylmercapto-, Trifluormethyl-, Nitro-, Nitril- oder Carbonestergruppen mit 1—4 Kohlenstoffatomen im Alkoholteil in Frage kommen,

und deren pharmazeutisch verwendbaren Salze eine den bekannten Chinolon- und Azachinoloncarbonsäuren überlegene antibakterielle Wirkung aufweisen.

Weiterhin wurde gefunden, daß man 7-Amino-1-cyclopropyl-4-oxo-1,4-dihydro-naphthyridin-3-carbonsäuren der Formel I erhält, wenn man Chinoloncarbonsäuren der Formel II (R = H)

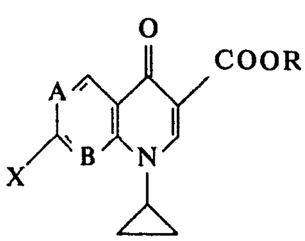

(II)

in welcher
A und B die oben angegebene Bedeutung haben und X für ein Halogenatom oder eine Alkylsulfonyl gruppe mit 1 bis 4 Kohlenstoffatomen steht,

mit Aminen der Formel II

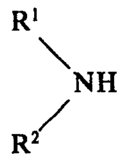

(III)

in welcher
$R^1$ und $R^2$ die oben angegebene Bedeutung haben,

umsetzt. Ferner kann man 7-Halogen-naphthyridin-3-carbon-säureester II (R=Alkyl) mit III, gegebenenfalls in Gegenwart eines Säurebinders, wie z. B. Triethylamin oder Pyridin, umsetzten und anschließend die erhaltene 7-Amino-naphthyridin-3-carbonsäureester alkalisch zu I verseifen.

Verwendet man bei der Umsetzung von II mit III beispielhaft 7-Chlor-1-cyclopropyl-4-oxo-1,4-dihydro-1,6-naphthyridin-3-carbonsäure und N-Methylpiperazin als Reaktanten, so kann der Reaktionsverlauf durch das folgende Formelschema wiedergegeben werden:

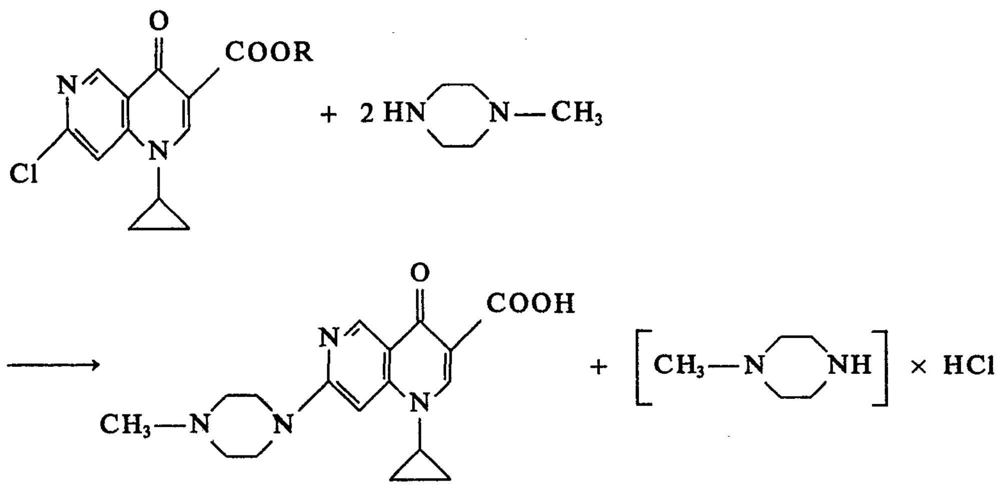

Die Ausgangsverbindungen II können auf folgende Weise hergestellt werden:

Man geht beispielsweise von in 6-Stellung durch X substituierten 4-Halogen-pyridin-3-carbonsäureestern der Formel IV aus, die mit $\beta$-Cyclopropylamino-propion-säureestern der Formel V, bevorzugt Methyl- oder Ethylester, die durch Umsetzung von entsprechenden Acrylsäureestern mit Cyclopropylamin leicht zugänglich sind, weitgehend selektiv unter Austausch des 4-ständigen Halogenatoms gegen den Aminrest zu den Monosubstitutionsprodukten der Formel VI um. Letztere gehen in Gegenwart einer starken Base, wie z. B. Kalium-t-butanolat oder Natriumhydrid durch Dieckmann-Cyclisierung in die Tetrahydro-naphthyridin-3-carbonsäureester der Formel VII über. Mit Brom oder Sulfurylchlorid und Triethylamin oder Pyridin als Dehydrohalogenierungsmittel erhält man aus VII die Carbonester der Formel VIII, die mit Alkali zu den Carbonsäuren der Formel II (R=H, A=N, B=CH) verseift werden können.

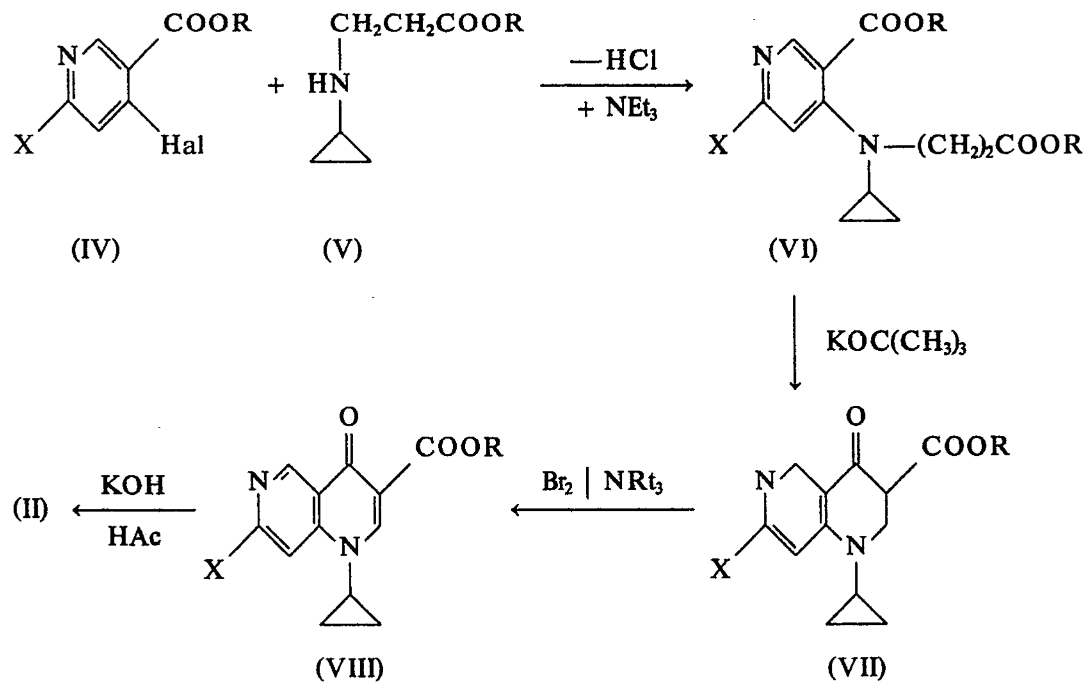


(IV) (V) (VI) (VII) (VIII) (II)

Als Verdünnungsmittel für die Reaktion II →I kommen vorzugsweise Äthanol, Dioxan, Toluol, DMF und Dimethylsulfoxid in Frage. Als Säurebinder können vorzugsweise Alkalicarbonate, Alkalihydroxide, oder tert. organische Basen wie z. B. Triethylamin, Pyridin usw. dienen.

Die Reaktionstemperaturen kann man in einem größerem Bereich variieren. Im allgemeinen arbeitet man zwischen etwa 20° und etwa 180° C, vorzugsweise zwischen 60° und 140° C.

Die Umsetzung kann bei Normaldruck, aber auch bei erhöhtem Druck, insbesondere bei gasförmigen und niedrig siedenden Aminen der Formel III durchgeführt werden. Im allgemeinen arbeitet man bei Drucken zwischen etwa 1 und etwa 100 bar, vorzugsweisse zwischen 1 und 10 bar.

Bei der Durchführung des Verfahrens setzt man auf 1 Mol Carbonsäure 1—5 Mol Amin, vorzugsweise 2—3 Mol Amin ein.

Als neue antibakerielle Wirkstoffe seien im einzelnen genannt:

7-Methylamino-, 7-Benzylamino-, 7-Pyrrolidino-, 7-Morpholino-, 7-Piperidino-, 7-Piperazino-, 7-(4-Methylpiperazino)-,7-(4-Benzylpiperazino)-, 7-(4-β-Hydroxyetylpiperazino)-, 7-(4-γ-Hydroxy-propylpiperazino)-, 7-(4-Formylpiperazino)-, 7-(4-Hydroxypiperidino)-1-cyclopropyl-4-oxo-1,4-dihydro-1,6-naphthyridin-3-carbonsäure und pharmazeutisch verträgliche Säureadditionssalze oder Alkalisalze dieser Verbindungen.

## Beispiel 1

7-(4-Methylpiperazino)-1-cyclopropyl-4-oxo-1,4-dihydro-1,6-naphthyridin-3-carbonsäure
(I, $R^1R^2N$ = 4-Methylpiperazino, A = N, B = CH).

Eine Suspension von 2,64 g 7-Chlor-1-cyclopropyl-4-oxo-1,4-dihydro-1,6-naphthyridin-3-carbonsäure und 2,5 g N-Methylpiperazin in 30 ml Äthanol wird 16 Stunden unter Rückfluß zum Sieden erhitzt. Man destilliert das Verdünnungsmittel im Vakuum ab, löst den Rückstand in 30 ml 1 N NaOH, filtriert und säuert mit 10%iger Salzsäure an. Der Niederschlag wird abgesaugt und mit Wasser und Äthanol gewaschen. Die Umkristallisation kann aus N-Dimethylformamid/Äthanol erfolgen. Man erhält 3,1 g (94% der theoretischen Ausbeute) 7-(4-Methylpiperazino)-1-cyclopropyl-4-oxo-1,4-dihydro-1,6-naphthyridin-3-carbonsäure vom Schmelzpunkt 326° C (Zers.) (Hydrochlorid).

## Beispiele 2 bis 10

Analog der Arbeitsweise im Beispiel 1 wurden die Carbonsäuren der Beispiele 2 bis 10 erhalten. Sie sind in der Tabelle 1 zusammengestellt. Die Bezifferung der Reste $R^1$ und $R^2$ beziehen sich auf die Formel I der Beschreibung.

Tabelle 1

| Beispiel Nr. | A | B | $R^1$ $R^2$ | Zersp. (°C) |
|---|---|---|---|---|
| 2 | N | CH | H<br>$-(CH_2)_2N(CH_2)_2-$ | 322 (Hydrochlorid) |
| 3 | N | CH | $-(CH_2)_2O(CH_2)_2-$ | 286 |
| 4 | N | CH | $-(CH_2)_2CH_2(CH_2)_2-$ | 297 |
| 5 | N | CH | $-CH_2CH_2CH_2CH_2-$ | 330 |
| 6 | N | CH | $-(CH_2)_2N(CH_2)_2-$<br>\|<br>$CH_2CH_2OH$ | 305 (Hydrochlorid) |
| 7 | N | CH | $-(CH_2)_2N(CH_2)_2-$<br>\|<br>$(CH_2)_3OH$ | 306 (Hydrochlorid) |
| 8 | N | CH | $-(CH_2)_2N(CH_2)_2-$<br>\|<br>CHO | 300 |
| 9 | N | CH | $-CH_2-CH-(CH_2)_3-$<br>\|<br>OH | 302 |
| 10 | N | CH | $-(CH_2)_2CH(CH_2)_2-$<br>\|<br>OH | 279 |
| 11 | CF | CH | H<br>$-(CH_2)_2N(CH_2)_2-$ | 256<br>306 (Hydrochlorid) |
| 12 | CH | N | $-(CH_2)_2N(CH_2)_2-$<br>\|<br>$CH_3$ | 279 |
| 13 | CH | N | $-(CH_2)_2N(CH_2)_2-$<br>H | 277 |
| 14 | CF | CH | $-(CH_2)_2N(CH_2)_2-$<br>\|<br>$CH_3$ | 249 |
| 15 | CF | CH | $-(CH_2)_4-$ | 323 |
| 16 | C—CN | N | H<br>$-(CH_2)_2N(CH_2)_2-$ | 335 (Hydrochlorid) |
| 17 | C—CN | N | $-(CH_2)_2N(CH_2)_2-$<br>\|<br>$CH_3$ | 295 (Hydrochlorid) |
| 18 | C—CN | N | $-(CH_2)_4-$ | 290 |
| 19 | CF | CH | $-(CH_2)_2N(CH_2)_2-$<br>\|<br>$C_2H_5$ | 306 (Hydrochlorid) |

Die als Ausgangsmaterial verwendete 7-Chlor-1-cyclopropyl-4-oxo-1,4-dihydro-1,6-naphthyridin-3-carbonsäure kann in einer mehrstufigen Reaktionsfolge z. B. ausgehend vom bekannten, 4,6-Dichlornicotinsäuremethylester (Recueil Trav. chim. Pays-bas. 69, 687 (1950) hergestellt werden.

1) 6-Chlor-4-(N-2-methoxycarbonylethyl-N-cyclopropyl-amino-pyridin-3-carbonsäuremethylester (VI, R = Methyl, X = Chlor:

Eine Lösung von 41,2 g 4,6-Dichlor-pyridin-3-carbonsäuremethylester in 150 ml Toluol wird unter Eiskühlung und Rühren in rascher Tropfenfolge mit einem Gemisch von 28,6 g β-Cyclopropylamino-propion-säuremethylester und 21 g Triethylamin bei 10—20°C versetzt. Man entfernt das Eisbad, rührt 1/2 Stunde bei Raumtemperatur und erhitzt 6 Stunden unter Rückfluß zum Sieden. Die erhaltene Suspension wäscht man mit Wasser, trocknet mit $Na_2SO_4$ und destilliert das Lösungsmittel im Vakuum ab. Es werden 59 g der Titelverbindung als braunes Öl erhalten.
Der als Reaktant verwendete β-Cyclopropylaminopropionsäuremethylester wurde wie folgt hergestellt:
Zu einer auf —60°C bis —70°C gekühlten Lösung von 57 g Cyclopropylamin in 150 ml Äthanol tropft man in ca. 3 Stunden 86 g auf —60°C gekühlten, frisch destillierten Acrylsäuremethylester. Dann läßt man über Nacht langsam auf Raumtemperatur kommen, destilliert das Lösungsmittel im Vakuum ab und fraktioniert anschließend. Bei 84—86°C/22 Torr gehen 95 g β-Cyclopropylaminopropionsäuremethylester über.

2) 7-Chlor-1-cyclopropyl-4-oxo-1,2,3,4-tetrahydro-1,6-naphthyridin-3-carbonsäuremethylester (VII, R = Methyl, X = Chlor):

59 g roher 6-Chlor-4-(N-2-methoxycarbonylethyl-N-cyclopropyl)-amino-pyridin-3-carbonsäuremethylester werden in 240 ml wasserfreiem Toluol gelöst und unter Rühren rasch mit 23 g Kalium-t-butanolat versetzt. Man läßt über Nach stehen, gibt 20 g Eisessig und 100 ml Wasser zu, trennt die Phasen, wäscht die Toluollösung nochmals mit Wasser, trocknet mit $Na_2SO_4$ und zieht das Toluol im Vakuum ab. Man erhält nach dem Umkristallisieren aus Methanol 18 g Carbonester vom Schmelzpunkt 155—157°C.

3) 7-Chlor-1-cyclopropyl-4-oxo-1,4-dihydro-1,6-naphthyridin-3-carbonsäuremethylester (VIII, R = Methyl, X = Chlor):

9,8 g des nach 2) hergestellten Tetrahydronaphthyridin-3-carbonsäuremethylesters wurden in 200 ml Methylenchlorid gelöst und unter Eiskühlung bei 10—15°C rasch tropfenweise mit einer Lösung von 5,9 g Brom in 40 ml $CH_2Cl_2$ versetzt. Dann wird noch 10 Minuten bei ~10°C gerührt, mit 8 g Triethylamin versetzt und das Eisbad entfernt. Man rührt 3 Stunden nach, wäscht zweimal mit Wasser, trocknet mit $Na_2SO_4$, destilliert das Lösungsmittel im Vakuum ab und kristallisiert den Rückstand aus DMF/Äthanol um. Man erhält 8,8 g 7-Chlor-1-cyclopropyl-4-oxo-1,4-dihydro-naphthyridin-3-carbonsäuremethylester vom Schmelzpunkt 272° —274°C (Zers.).

4) 7-Chlor-1-cyclopropyl-4-oxo-1,4-dihydro-1,6-napthyridin-3-carbonsäure (II, R = H, A = N, B = CH, X = Chlor):

27,85 g des nach 3) hergestellten Esters werden mit einer Lösung von 5,7 g Ätzkali in 300 ml Wasser versetzt. Unter Rühren erhitzt man 30 Minuten auf 85—95°C, filtriert die erhaltene Lösung bei Raumtemperatur und säuert mit Eisessig an. Der Niederschlag wird abgesaugt, mit Wasser gewaschen und im Vakuumtrockenschrank über Calciumchlorid getrocknet. Man erhält 20 g reine 7-Chlor-1-cycloprophyl-4-oxo-1,4-dihydro-1,6-naphthyridin-3-carbonsäure vom Schmelzpunkt 226—227°C.

Es wurde weiterhin gefunden, daß die erfindungsgemäßen Verbindungen hervorragende antimikrobielle Eigenschaften besitzen.
Insbesondere sind sie breit bakteriostatisch und bakterizid wirksam gegen grampositive Bakterien, wie Staphylokokken und Streptokokken, und gramnegative Bakterien wie Escherichia, Proteus, Providencia, Enterobacter, Klebsiella, Salmonella und Pseudomonas. Die verbesserte antibakterielle Wirkung der erfindungsgemäßen neuen Verbindungen wird besonders deutlich an dem Beispiel 1, das sich im Vergleich zu 2-Piperazino-8-ethyl-5-ox-5,8-dihydro-pyrido [2,3-d] pyrimidin-6-carbonsäure (»Pipemidin Säure«) oder der bekannten 1-Ethyl-7-methyl-4-naphthyridon (1,8)-3-carbonsäure [»Nali

dixinsäure«; Ehrhart/Ruschig, Arzneimittel, Band 2: Chemotherapeutika, Verlag Chemie 1968, Seite 1568] in vitro und in vivo bei Staphylokokken, Escherichia coli, Proteus, Klebsiella, Pseudomonas als weit überlegen erwies.

Die verbesserte breite antibakterielle Wirksamkeit der erfindungsgemäßen Verbindungen ermöglicht ihren Einsatz als Wirkstoffe sowohl in der Human- als auch in der Veterinärmedizin, wobei sie sowohl zur Verhütung als auch zur Behandlung von systemischen oder lokalen bakteriellen Infektionen insbesondere der Harnwege verwendet werden können. Die erfindungsgemäßen Verbindungen können weiterhin auch als Futterzusatzmittel zur Förderung des Wachstums und zur Verbesserung der Futterauswertung in der Tierhaltung, insbesondere bei der Haltung von Mastvieh, verwendet werden. Die Applikation der Wirkstoffe erfolgt dann vorzugsweise über das Futter und/oder das Trinkwasser.

Die vorliegende Erfindung betrifft weiterhin Mittel, die die neuen erfindungsgemäßen Verbindungen enthalten. Hierzu gehören beispielsweise Futtermittelkonzentrate für die Tierhaltung, die in üblicher Weise neben den Wirkstoffen auch Vitamine und/oder Mineralsalze enthalten können oder pharmazeutische Zubereitungen.

Die Erfindung betrifft bevorzugt antibakteriell wirksame Mittel, die Verbindungen der Formel I enthalten. Die Erfindung betrifft besonders bevorzugt solche antibakteriell wirksame Mittel, die Verbindungen der Formel I oder deren Alkali- oder Erdalkalisalze enthalten.

Die erfindungsgemäßen pharmazeutischen Zubereitungen enthalten neben den neuen erfindungsgemäßen Verbindungen in üblicher Weise nicht toxische, inerte pharmazeutisch geeignete Trägerstoffe. Solche pharmazeutisch geeignete Trägerstoffe sind beispielsweise Füll- und Streckmittel, Bindemittel, Feuchthaltemittel, Lösungsverzögerer, Resorptionsbeschleuniger, Netzmittel, Adsobtionsmittel oder Gleitmittel, die feste, halbfeste oder flüssige Konsistenz haben können. Solche pharmazeutisch geeignete Trägerstoffe sind dem Fachmann bekannt.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotionen, Puder und Sprays genannt. Die Herstellung dieser Zubereitungen geschieht nach bekannten Methoden in üblicher Weise, beispielsweise durch Mischen des neuen erfindungsgemäßen Wirkstoffes mit den üblichen Träger- und Zusatzstoffen. Der Wirkstoff soll in den aufgeführten pharmazeutischen Zubereitungen in einer Konzentration von etwa 0,1 bis 99,5, vorzugsweise von etwa 0,5 bis 95 Gew.-% der Gesamtmischung vorhanden sein.

Die Bereitstellung neuer Bakterizide zur Bekämpfung von Bakterien, die gegen bekannte Bakterizide resistent sind, ist eine Bereicherung des Standes der Technik.

Minimale Hemmkonzentrationen mcg/ml im Agarverdünnungstest*)

| | Verbindungen von Beispiel 1 | Beispiel 11 | Pipemidinsäure | Nalidixinsäure |
|---|---|---|---|---|
| Escherichia coli | | | | |
| T 7 | 0,25 | $\leqq 0{,}015$ | 2 | 1 |
| 455/7 | 128 | 1,0 | 128 | >256 |
| 103400 | 0,25 | | 1 | 2 |
| Salmonella 683 | 0,5 | | 2 | 4 |
| Klebsiella 63 | 1 | | 2 | 4 |
| Pseudomonas 7167 | 8 | 0,5 | 16 | 64 |
| Proteus 8228 | 2 | | 4 | 8 |

*) Denley-Multipoint-Inokulationsverfahren.

**Patentansprüche**

1. 7-Amino-1-cyclopropyl-4-oxo-1,4-dihydro-naphthyridin-3-carbonsäuren der allgemeinen Formel I

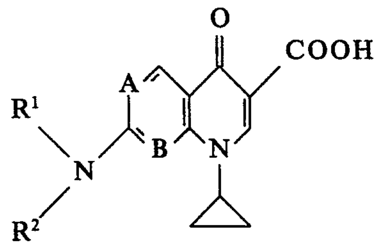

(I)

in welcher

A Stickstoff oder $CR^3$ sein kann, wobei $R^3$ Wasserstoff, Nitro, Halogen, bevorzugt Fluor oder Chlor, oder eine Nitril-, Carbonamid-, Carboxyl- oder Estergruppe sein kann und

B Stickstoff oder C-H darstellt und A und B nicht gleichzeitig Stickstoff sein können sowie

$R^1$ und $R^2$ gleich oder verschieden sind und für Wasserstoff, einen verzweigten oder unverzweigten Alkyl-, Alkenyl- oder Alkinylrest mit 1—12 Kohlenstoffatomen stehen, der gegebenenfalls durch Hydroxylgruppen, Alkoxy-, Alkylmercapto- oder Dialkylamino-Gruppen mit 1—3 Kohlenstoffatomen in jedem Alkylrest, die Nitril- sowie eine Alkoxycarbonyl-Gruppe mit 1—4 Kohlenstoffatomen im Alkoholteil substituiert sein kann, ferner Cycloalkyl mit 3—6 Kohlenstoffatomen bedeutet und weiterhin gemeinsam mit dem Stickstoffatom, das sie substituieren und gegebenenfalls einem Heteroatom wie z. B. Sauerstoff, Schwefel oder $NR^4$ einen 3—7-gliedrigen Ring bilden, der ein- oder mehrfach durch Alkyl- oder Alkenylgruppen mit 1—6 Kohlenstoffatomen, Hydroxyl-, Alkoxy- und Alkylmercaptogruppen mit 1—3 Kohlenstoffatomen, eine Alkoxycarbonylgruppe mit 1—4 Kohlenstoffatomen im Alkoholteil, die Nitrilgruppe sowie einen Phenylrest substituiert sein kann und ferner eine Doppelbindung besitzen kann und

$R^4$ Wasserstoff, eine verzweigte oder unverzweigte Alkyl-, Alkenyl- oder Alkinylgruppe mit 1—6 Kohlenstoffatomen, die gegebenenfalls durch Hydroxyl, Alkoxy-, Alkylmercapto- und Dialkylaminogruppe mit 1—3 Kohlenstoffatomen für einen Alkylrest, die Alkoxycarbonylgruppe mit 1—4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, sowie eine gegebenenfalls substituierte Phenyl- oder Naphthylgruppe oder ein heterocyclischer Rest wie beispielsweise einen Pyridin-, Pyrimidin-, Thiazol- oder Benzthiazolkern darstellt, ferner eine gegebenenfalls durch einen Phenylrest substituierte Alkoxycarbonylgruppe mit 1—4 Kohlenstoffatomen im Alkoholteil, ein Alkanoylrest mit 1—6 Kohlenstoffatomen, ein Benzoylrest, ein gegebenenfalls substituierter Cl—C6-Alkyl- oder Rhenylsulfonylrest sowie ein gegebenenfalls substituierter Aminosulfonylrest bedeutet, wobei als Substituenten von Phenyl- und Naphthylgruppen Halogen, Alkyl-, Alkoxy- oder Alkylmercaptogruppen mit 1—3 Kohlenstoffatomen, Phenyloxy-, Phenylmercapto, Trifluormethyl-, Nitro-, Nitril- oder Carbonestergruppen mit 1—4 Kohlenstoffatomen im Alkoholteil in Frage kommen,

und deren pharmazeutisch verwendbaren Salze.

2. 6-Fluor-7-piperazino-1-cyclopropyl-4-oxo-1,4-diphydro-chinolin-3-carbonsäure.

3. 7-(4-Methylpiperazino)-1-cyclopropyl-4-oxo-1,4-dihydro-naphthyridin-3-carbonsäure.

4. 7-Piperazino-1-cyclopropyl-4-oxo-1,4-dihydro-naphthyridin-3-carbonsäure.

5. 7-(4-Hydroxyethylpiperazino)-1-cyclopropyl-4-oxo-1,4-dihydro-naphthyridin-3-carbonsäure.

6. 7-(4-Formylpiperazino)-1-cyclopropyl-4-oxo-1,4-dihydro-naphthyridin-3-carbonsäure.

7. Verfahren zur Herstellung von 7-Amino-1-cyclopropyl-4-oxo-1,4-dihydro-naphthyridin-3-carbonsäuren der allgemeinen Formel I in Anspruch 1, dadurch gekennzeichnet, daß man

(a) Naphthyridon-3-carbonsäuren der allgemeinen Formel II (R = H)

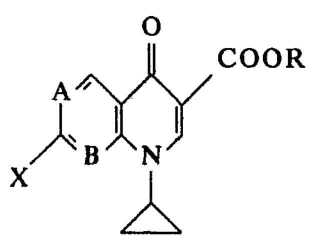

(II)

in welcher
A und B die oben angegebene Bedeutung haben und
X für ein Halogenatom oder eine Alkylsulfonylgruppe mit 1—4 Kohlenstoffatomen steht,

mit Aminen der allgemeinen Formel III

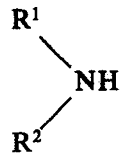

(III)

in welcher
$R^1$ und $R^2$ die oben angegebene Bedeutung haben,

umsetzt, oder

(b) 7-Halogen-naphthyridon-3-carbonsäureester der allgemeinen Formel II (R = Alkyl) mit Aminen der allgemeinen Formel III gegebenenfalls in Gegenwart eines Säurebinders, wie z. B. Triethylamin oder Pyridin, umsetzt und anschließend die erhaltenen 7-Amino-naphthyridin-3-carbonsäureester alkalisch verseift.

8. Arzneimittel, gekennzeichnet durch einen Gehalt an 7-Amino-1-cyclopropyl-4-oxo-1,4-dihydro-naphthyridin-3-carbonsäuren gemäß Anspruch 1.

9. Verfahren zur Herstellung von antibakteriellen Mitteln, dadurch gekennzeichnet, daß man 7-Amino-1-cyclopropyl-4-oxo-1,4-dihydro-naphthyridin-3-carbonsäuren gemäß Anspruch 1 mit inerten, nicht-toxischen pharmazeutisch geeigneten Trägerstoffen vermischt.

**Claims**

1. 7-Amino-1-cyclopropyl-4-oxo-1,4-diphydro-naphthyridine-3-carboxylic acids of the general formula I

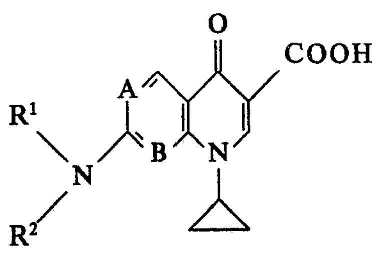

(I)

in which

A can be nitrogen or $CR^3$

wherein

$R^3$ can be hydrogen, nitro, halogen, preferably fluorine or chlorine, or a nitrile, carboxamide, carboxyl or ester group, and

B represents nitrogen or C—H, and A and B cannot simultaneously be nitrogen, and

$R^1$ and $R^2$ are identical or different and represent hydrogen or a branched or straight-chain alkyl, alkenyl or alkinyl radical which has 1—12 carbon atoms and can optionally be substituted by hydroxyl groups, alkoxy, alkylmercapto or dialkylamino groups with 1—3 carbon atoms in each alkyl radical, the nitrile group or an alkoxy-carbonyl group with 1—4 carbon atoms in the alcohol part of furthermore denotes cycloalkyl with 3—6 carbon atoms, or, together with the nitrogen atom which they substitute and, if appropriate, a hetero-atom, such as, for example, oxygen or sulphur, or $NR^4$, form a 3-membered to 7-membered ring which can be monosubstituted or polysubstituted by alkyl or alkenyl groups with 1—6 carbon atoms, hydroxyl groups, alkoxy or alkylmercapto groups with 1—3 carbon atoms, an alkoxycarbonyl group with 1—4 carbon atoms in the alcohol part, the nitrile group or a phenyl radical and can furthermore possess a double bond, and

$R^4$ represents hydrogen, or a branched or straightchain alkyl, alkenyl or alkinyl group which has 1—6 carbon atoms and can optionally be substituted by hydroxyl, an alkoxy, alkylmercapto or dialkylamino group with 1—3 carbon atoms per alkyl radical or the alkoxycarbonyl group with 1—4

carbon atoms in the alcohol part, or represents a phenylalkyl group which is optionally substituted in the phenyl radical and has up to 4 carbon atoms in the aliphatic part, or an optionally substituted phenyl or naphthyl group or a heterocyclic radical, such as, for example, a pyridine, pyrimidine, thiazole or benzothiazole nurcleus, or denotes an alkoxycarbonyl group which is optionally substituted by a phenyl radical and has 1—4 carbon atoms in the alcohol part, an alkanoyl radical with 1—6 carbon atoms, a benzoyl radical, an optionally substituted C1—C6-alkyl or phenylsulphonyl radical or an optionally substituted aminosulphonyl radical, possible substituents of phenyl and naphthyl groups being halogen, alkyl, alkoxy or alkylmercapto groups with 1—3 carbon atoms, phenyloxy, phenylmercapto, trifluoromethyl, nitro, nitrile or carboxylic ester groups with 1—4 carbon atoms in the alcohol part,

and pharmaceutically usable salts thereof.

2. 6-Fluoro-7-piperazino-1-cyclopropyl-4-oxo-1,4-dihydro-quinoline-3-carboxylic acid.

3. 7-(4-Methylpiperazino)-1-cyclopropyl-4-oxo-1,4-dihydro-naphthyridine-3-carboxylic acid.

4. 7-Piperazino-1-cyclopropyl-4-oxo-1,4-dihydro-naphthyridine-3-carboxylic acid.

5. 7-(4-Hydroxyethylpiperazino)-1-cyclopropyl-4-oxo-1,4-dihydro-naphthyridine-3-carboxylic acid.

6. 7-(4-Formylpiperazino)-1-cyclopropyl-4-oxo-1,4-dihydro-naphthyridine-3-carboxylic acid.

7. Process for the preparation of 7-amino-1-cycloprophyl-4-oxo-1,4-dihydro-naphthyridine-3-carboxylic acids of the general formule I in Claim 1, characterised in that

(a) naphthyridone-3-carboxylic acids of the general formula II (R = H)

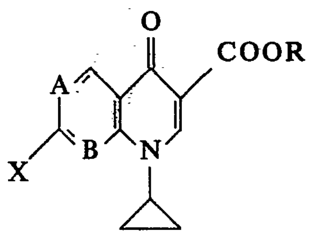

(II)

in which
A and B have the abovementioned meaning and
X represents a halogen atom or an alkylsulphonyl group with 1—4 carbon atoms,

are reacted with amines of the general formula III

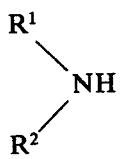

(III)

in which
$R^1$ and $R^2$ have the abovementioned meaning,
or

(b) 7-halogeno-naphthyridone-3-carboxylic acid esters of the general formula II (R = alkyl) are reacted with amines of the general formula III, if appropriate in the presence of an acid-binding agent, such as, for example, triethylamine or pyridine, and the resulting 7-amino-naphthyridine-3-caroxylic acid esters are then hydrolysed under alkaline conditions.

8. Medicaments, characterised in that they contain 7-amino-1-cyclopropyl-4-oxo-1,4-dihydro-naphthyridine-3-carboxylic acids according to Claim 1.

9. Process for the preparation of antibacterial agents, characterised in that 7-amino-1-cyclopropyl-4-oxo-1,4-dihydro-naphthyridine-3-carboxylic acids according to Claim 1 are mixed with inert, non-toxic pharmaceutically suitable excipients.

**Revendications**

1. Acides 7-amino-1-cyclopropyl-4-oxo-1,4-dihydro-naphtyridine-3-carboxyliques de formule générale I:

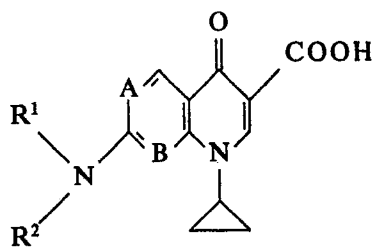

(I)

dans laquelle

A peut être un atome d'azote ou $CR^3$, $R^3$ pouvant être un atome d'hydrogène, un groupe nitro, un atome d'halogène, de préférence, un atome de fluor ou un atome de chlore, ou encore un groupe nitrile, carbonamide, carboxy ou ester, et

B représente un atome d'azote ou CH, A et B ne pouvant être simultanément un atome d'azote, et

$R^1$ et $R^2$ sont idenetiques ou différents et représentent chacun un atome d'hydrogène, un groupe alkyle, un groupe alcényle ou un groupe alcynyle ramifié ou non ramifié, contenant 1 à 12 atomes de carbone et pouvant être éventuellement substitué par des groupes hydroxy, des groupes alcoxy, alkylmercapto ou dialkylamino contenant 1 à 3 atomes de carbone dans chaque groupe alkyle, le groupe nitrile, ainsi que par un groupe alcoxy-carbonyle contenant 1 à 4 atomes des carbone dans la fraction alcool; ils représentent également un groupe cycloalkyle contenant 3 à 6 atomes de carbone et, en outre, ensemble avec l'atome d'azote qu'ils substituent et éventuellement un hétéro-atome tel que, par exemple, un atome d'oxygène, un atome de soufre ou $NR^4$, ils forment un noyau triangulaire à heptagonal qui peut être substitué une ou plusieurs fois par des groupes alkyle ou alcényle contenant 1 à 6 atomes de carbone, par des groupes hydroxy-, alcoxy- et alkyl-mercapto contenant 1 à 3 atomes de carbone, par un groupe alcoxycarbonyle contenant 1 à 4 atomes de carbone dans la fraction alcool, par le groupe nitrile, ainsi que par un groupe phényle et, en outre, ils peuvent comporter une double liaison, et

$R^4$ représente un atome d'hydrogène, un groupe alkyle, alcényle ou alcynyle ramifié ou non ramifié contenant 1 à 6 atomes de carbone et qui peut éventuellement être substitué par un groupe hydroxy, alcoxy, alkyl-mercapto et dialkylamino contenant 1 à 3 atomes de carbone pour un radical alkyle, et par un groupe alcoxycarbonyle contenant 1 à 4 atomes de carbone dans la fraction alcool, un groupe phénylalkyle éventuellement substitué dans le radical phényle et contenant jusqu'à 4 atomes de carbone dans la fraction aliphatique, ainsi qu'un groupe phényle ou naphtyle éventuellement substitué ou un radical hétérocyclique tel que, par exemple, un noyau pyridine, pyrimidine, thiazole ou benzthiazole, de même qu'un groupe alcoxycarbonyle éventuellement substitué par un groupe phényle et contenant 1 à 4 atomes de carbone dans la fraction alcool, un radical alcanoyle contenant 1 à 6 atomes de carbone, un radical benzoyle, un radical alkyl en $C_1$-$C_6$-ou phénylsulfonyle éventuellement substitué, de même qu'un radical aminosulfonyle éventuellement substitué et, comme substituants des groupes phényle et naphtyle, on envisage les atomes d'halogènes, les groupes alkyle-, alcoxy- ou alkylène-mercapto contenant 1 à 3 atomes de carbone, les groupes phényloxy, phényl-mercapto, trifluorométhyle, nitro, nitrile ou ester carboxylique contenant 1 à 4 atomes de carbone dans la fraction alcool,

ainsi que leurs sels pharmaceutiquement utiles.

2. L'acide 6-fluoro-7-pipérazino-1-cyclopropyl-4-oxo-1,4-dihydro-quinoléine-3-carboxylique.

3. L'acide 7-(4-méthylpipérazino)-1-cyclopropyl-4-oxo-1,4-diphydro-naphtyridine-3-carboxylique.

4. L'acide 7-pipérazino-1-cyclopropyl-4-oxo-1,4-dihydro-naphtyridine-3-carboxylique.

5. L'acide 7-(4-hydroxyéthylpipérazino)-1-cyclopropyl-4-oxo-1,4-dihydro-naphtyridine-3-carboxylique.

6. L'acide 7-(4-formylpipérazino)-1-cyclopropyl-4-oxo-1,4-dihydro-naphtyridine-3-carboxylique.

7. Procédé de préparation d'acides 7-amino-1-cyclopropyl-4-oxo-1,4-dihydronaphtyridine-3-carboxyliques de formule générale I suivant la revendication 1, caractérisé en ce que:

(a) on fait réagir des acides naphtyridone-3-carboxyliques de formule générale II (R = H):

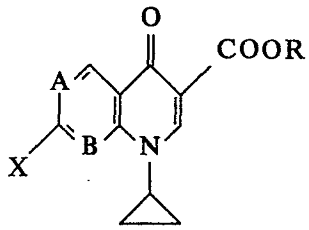

(II)

dans laquelle
A et B ont les significations indiquées ci-dessus et
X représente un atome d'halogène ou un groupe alkylsulfonyle contenant 1 à 4 atomes de carbone,

avec des amines de formule générale III:

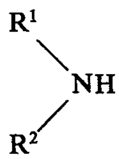

(III)

dans laquelle
$R^1$ et $R^2$ ont les significations indiquées ci-dessus,
ou

(b) on fait réagir des esters d'acides 7-halogéno-naphtyridone-3-carboxyliques de formule geénérale II (R = alkyle) avec amines de formule générale III éventuellement en présence d'un fixateur d'acide tel que, par exemple, la triéthylamine ou la pyridine, puis on saponifie, par voie alcaline, les esters d'acides 7-amino-naphtyridine-3-carboxyliques obtenus.

8. Médicaments, caractérisés en ce qu'ils contiennent des acides 7-amino-1-cyclopropyl-4-oxo-1,4-dihydro-naphtyridine-3-carboxyliques suivant la revendication 1.

9. Procédé de préparation d'agents antibactériens, caractérisé en ce qu'on mélange des acides 7-amino-1-cyclopropyl-4-oxo-1,4-dihydro-naphtyridine-3-carboxyliques suivant la revendication 1 avec des substances supports inertes, non toxiques et pharmaceutiquement appropriées.